# EUROPEAN PATENT APPLICATION

(11) **EP 1 407 720 A1**
(43) Date of publication of application: **14.04.2004**
(21) Application number: 04075012.7
(22) Date of filing: 03.05.1996
(51) Int. Cl.: A61B 18/14, A61B 18/12, A61N 5/04

(54) **Apparatus for skin resurfacing**

(30) Priority: 05.05.1995 US 435822; 05.05.1995 US 435544; 05.01.1996 US 583815
(62) Divisional of application: 96914574.7
(71) Applicant: Thermage, Danville, California 94526 (US)
(72) Inventor: Knowlton, Edward W., Danville California 94506 (US)
(74) Representative: Tanner, James Percival

(57) **Abstract**

A skin resurfacing apparatus delivers thermal energy through an external skin surface to an underlying collagen containing tissue. Thermal delivery means are provided and include an interface surface configured to conform to the exterior skin layer surface. Electrode means are coupled to the thermal delivery means and configured to transfer thermal energy through the interface surface and the skin layer surface to the underlying collagen tissue. Thermal energy control means are coupled to the thermal delivery means and configured to provide sufficient thermal energy from the electrode means to contract the underlying collagen tissue with no deeper than a first degree burn formed on the exterior skin layer surface. Cabling means are coupled to the electrode means.

## Description

### BACKGROUND OF THE INVENTION

### Field of the Invention

This invention relates generally to a method and apparatus for shrinking collagen containing tissue, and more particularly to a method and apparatus to shrink collagen containing tissue while creating no more than a first degree burn on an external surface.

### Description of Related Art

The skin is composed of two basic elements, the epidermis and the underlying dermis. The underlying dermis provides the main structural support of the skin. The epidermis contains the epithelial cells and pigment forming cells called melanocytes. The dermis varies in thickness throughout the body. For instance, the skin is 25 times thicker on the back than on the eyelid.

The dermis is composed mainly of an extracellular protein called collagen. Collagen exists as a triple helix with three polypeptide chains that are connected with heat labile and heat stable chemical bonds. When collagen is heated, alterations in the physical properties of this protein occur at a characteristic temperature. This structural transition occurs at a specific shrinkage temperature.

The phenomenon of thermal shrinkage of collagen begins with a denaturization of the triple helix of the collagen molecule. Thermal energy severs the heat labile bonds that stabilize the triple stranded helix. As a result, the longitudinal axis of the molecule contracts. Partial denaturization of collagen tissue occurs in second degree burns and is typically applied as a standard thermal gradient that is hotter on the surface and cooler in the underlying dermis. In burn patients, partial denaturization of dermal collagen provides a tightening effect on the skin. By applying a reverse thermal gradient which cools the surface of the skin while heating the underlying collagen-containing layers, contraction of collagen in the absence of a second degree burn (and its inherent blistering and pigmentary irregularities) is possible. Because collagen is found in tendon, bone, cartilage and all other connective tissue throughout the body, reverse thermal gradient contraction of collagen can have many applications.

The selective induction of the basic wound healing process serves as the basis for the second major application of thermal shrinkage of collagen. In higher developed animal species, the wound healing response to injury involves an initial inflammatory process that subsequently leads to the deposition of scar tissue. The initial inflammatory response consists of the infiltration by white blood cells or leukocytes that dispose of cellular debris. Forty-eight hours later, proliferation of fibroblasts at the injured site occurs. These cells then produce scar collagen that functions as the main support structure of a healed wound. The deposition and subsequent remodeling of this nascent scar collagen provides the means to alter the consistency and geometry of soft tissue for both aesthetic and reconstructive purposes.

There exists an aesthetic need to contract skin without the scars, surgical risks or pigmentary side effects of commonly employed technique. These techniques include surgical resection of skin and the use of lasers and chemical peels to achieve a tighter, more youthful skin appearance. Understandably, many patients are hesitant to subject themselves to these procedures, even though an overall aesthetic improvement is likely.

Skin resection procedures are limited in their application due to inherent scars. With face-lift procedures, scars can be hidden around the contour of the ear, thus providing an acceptable trade-off between the surgical scar and the aesthetic improvement. Surgical resection of skin on the hips, thighs, arms, knees and legs, however, provides only a modest improvement with fairly unsightly scarring. In addition, patients must undergo a post-operative phase of healing that may be both painful and inconvenient. Other risk factors, such as bleeding and infection, may prolong healing.

Liposuction is effective at removing fat in some areas, however, it does not tighten the skin envelope. Skin resurfacing techniques that secondarily tighten excess skin (such as laser and chemical peels) employ a standard thermal gradient that requires burning off the superficial skin as a second degree burn. The thermal effects of collagen contraction in the deeper dermis occur, but require a painful healing phase due to the second degree burn. These modalities depend upon reepithelialization with cell migration from the skin appendages. This process of reepithelialization is similar to the healing of any thermal burn and is more likely to cause pigmentary irregularities due to the destruction of melanocytes in the epidermis.

Adipose tissue, more commonly known as fat, is formed of cells containing stored lipid. Adipose tissue is often subdivided into small loculations by connective collagen tissue serving as the fibrous septae.

There exists a need for skin tightening without damaging the melanocytes and other epithelial cells, or without surgical intervention. There is a further need for non-surgically removing adipose tissue without damaging the melanocytes and other epithelial cells.

There exists a need for subcutaneously contracting of collagen without surgical scarring or pigmentary side effects of more invasive techniques. There is a further need for subcutaneously inducing the formation and contraction of scar collagen in a selected tissue site while creating no deeper than a second degree burn on the surface of the selected tissue site.

### SUMMARY OF THE INVENTION

An object of the present invention to provide a method and apparatus for tightening skin without substantially damaging the melanocytes and other epithelial cells.

Another object of the invention is provide a method and apparatus to deliver sufficient thermal energy to reduce loculations of fat without substantially effecting the melanocytes and other epithelial cells.

A further object of the present invention is to provide a method and apparatus for contour sculpture by denaturing collagen in fibrous septae tissue.

Yet another object of the present invention to provide a method for thermal remodelling and contraction of collagen without surgical scarring or pigmentary side effects.

Another object of the present invention is to provide a method for inducing the formation and contraction of scar collagen.

A further object of the present invention is to provide a method for inducing the formation and contraction of bony callus in periosteum tissue.

Still another object of the present invention is to provide a method for contracting collagen tissue no deeper than a second degree burn formed on a tissue surface overlying the contracted collagen tissue, and preferably no deeper than a first degree burn.

These and other objects of the invention are achieved in an apparatus for delivering thermal energy through an external skin surface to an underlying collagen containing tissue. Thermal delivery means are provided and include an interface surface configured to conform to the exterior skin layer surface. Electrode means are coupled to the thermal delivery means and configured to transfer thermal energy through the interface surface and the skin layer surface to the underlying collagen tissue. Thermal energy control means are coupled to the thermal delivery means and configured to provide sufficient thermal energy from the electrode means to contract the underlying collagen tissue with no deeper than a first degree burn formed on the exterior skin layer surface. Cabling means are coupled to the electrode means.

In another embodiment, an apparatus is provided for applying radiant energy through the skin to an underlying subcutaneous layer, and deeper soft tissue layers, such as the muscle and overlying fascia, include loculations of fat with fibrous septae made of collagen tissue. Application of the radiant energy creates a desired contour effect without substantially modifying the melanocytes and other epithelial cells in the epidermis. The apparatus includes a membrane that conforms a contacting exterior surface of the membrane to a skin layer. One or more thermal electrodes are positioned in the membrane and create a reverse thermal gradient from the skin layer to the underlying collagen tissue. A focussing element focuses thermal energy to the underlying collagen tissue. The focussing element and the electrolytic solution create a reverse thermal gradient from the skin to the collagen tissue. A thermal power source is coupled to the thermal electrodes.

In another embodiment, a method is provided for forming and contracting scar collagen below a tissue surface in a selected tissue site. An electromagnetic energy apparatus is provided and includes an electromagnetic energy source and a delivery device. The delivery device is positioned on the tissue surface. Electromagnetic energy is produced from the electromagnetic energy source and delivered through the tissue surface to the selected tissue site for a sufficient time to induce scar collagen formation in the selected tissue site. No deeper than a second degree burn is formed on the tissue surface. The scar collagen is then contracted. This method is particularly useful in soft tissue sites that are devoid or deficient in collagen.

In another embodiment, a method is disclosed for forming callus deposition in a selected periosteum tissue site. An electromagnetic energy apparatus is provided and includes an electromagnetic energy source and a delivery device. The delivery device is positioned on a tissue surface of the selected periosteum tissue site. Electromagnetic energy is produced from the electromagnetic energy source. Electromagnetic energy is transcutaneously delivered from the delivery device, through the tissue surface, and to the selected periosteum tissue site for a sufficient time to induce callus formation in the selected periosteum tissue site. After scar collagen formation the callus is then contracted.

Suitable applications for the methods of the present invention include but are not limited to, tightening and firming soft tissue, unstable joints due to collateral ligament laxity, the treatment of unstable spinal column disorders, treatment of weaknesses of the abdominal wall, treatment of other connective tissues, esophageal hernia with reflux, urinary incontinence in women, dysdynamic segments of the myrocardium and other aneurysmal dilatations of the vessel, sleep apnea, laxity and wrinkling of the skin, and the like.

Wrinkling of the skin occurs as a consequence of inadequate support of the epidermis. The induction of scar collagen deposition is used for the treatment of wrinkles. Improved skin turgor is accomplished by first replenishing the collagen matrix that has been lost with aging. Following the deposition of nascent scar collagen in the dermis, contraction of collagen with a reverse thermal gradient corrects wrinkling of the skin without resorting to resurfacing techniques that require the application of a standard thermal gradient burn to the skin. This is achieved without undergoing a lengthy post-operative healing process. Bleeding and infection is reduced. Second degree bums to the superficial skin are minimized. The melanocytes are not damaged and pigmentary irregularities are avoided.

One apparatus used to create the reverse thermal gradient is a combined heating pad that has both cooling elements and electromagnetic delivery devices. The heating pad is configured to the topography of the treatment area and is incorporated into an elastic garment. Partial denaturization of collagen with contraction of skin is achieved with each treatment. Thermal transducers measure the surface temperature of the treatment area to avoid blistering. In one embodiment the deeper dermis is heated to above 65 degrees for collagen contraction. The temperature can vary depending on local tissue factors. Sequential treatments are designed to allow for more precision of the end result. Areas of application are not confined by requirements to either hide surgical incisions or transition along aesthetic boundaries.

Because scarring and pigmentary irregularities are avoided, skin or other tightening occurs in areas previously considered "off-limits" to standard methods of surgical resection, laser and chemical resurfacing. Skin tightening with a reverse thermal gradient contraction of collagen can correct areas including but not limited to the thighs, knees, arms, back and hips without unsightly scarring of standard techniques. In addition, areas previously corrected by aesthetic procedures, such as face and neck lifts, can be corrected without requiring surgery or the typical incisions around the ear. Elastosis or stretching of the abdominal skin from pregnancy can be corrected without the extensive incision of an abdominoplasty. The method of the present invention can also be used for mastopexies or breast uplifts.

The fibrous septae in subcutaneous fat layers can be contracted to tighten the soft tissue. Along with the extracellular effects of collagen, intracellular effects upon the fat cell, or lipocyte, by thermal induction cause a net reduction of fat in the lipocyte which achieves a net reduction in volume of the treated area. A second thermal device is used in tandem with the initial thermal device to achieve liposculpture of the treated area. The second device can be designed with a convergent lens that is focused at the appropriate level on the subcutaneous tissue.

A variety of electromagnetic energy sources can be employed. Suitable energy sources include but are not limited to RF, microwave, ultrasound and the like. In one embodiment, the preferred energy source is RF.

### BRIEF DESCRIPTION OF THE FIGURES

Fig. 1 is a perspective view of an apparatus for applying electromagnetic energy through the skin in order to cause a partial denaturization of collagen tissue, resulting in a tightening of the skin.
Figure 2 is a cross-sectional view of the skin and underlying tissue.
Figure 3 is a schematic representation of the collagen network.
Fig. 4 is a schematic diagram of an apparatus for applying electromagnetic energy to underlying subcutaneous layers or deeper soft tissue layers to create a desired contour effect by partially denaturing collagen tissue, and without substantially modifying melanocytes and other epithelial cells in the epidermis.

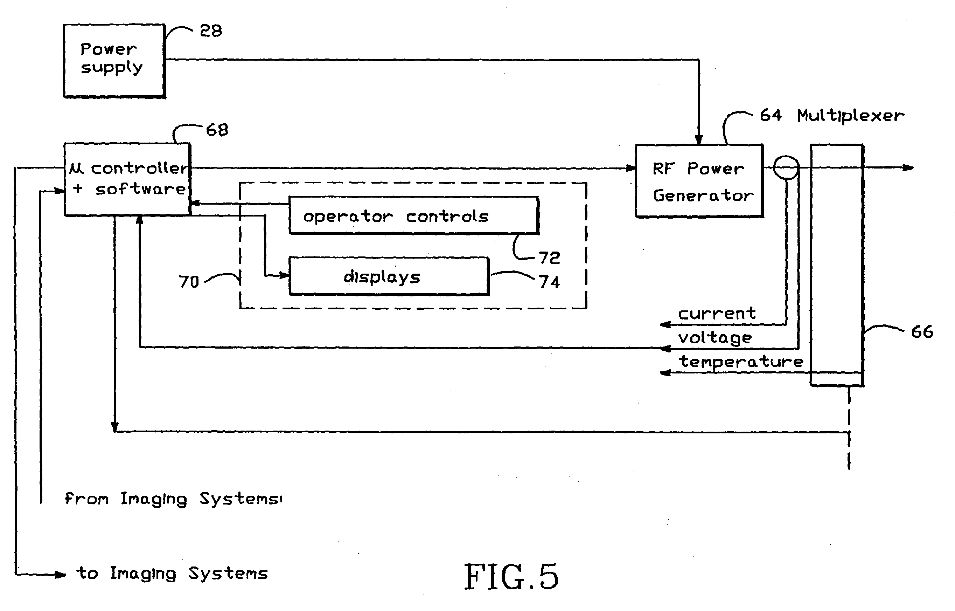

Figure 5 is a block diagram of an RF system which can be utilized with the present invention.
Figure 6 is a block diagram of processing circuit of one embodiment of the invention.

### DETAILED DESCRIPTION

For purposes of this specification, the following definitions apply.

Pre-existing collagen is the protein substance normally present in the white fibers (collagenous fibers) of skin, tendon, bone cartilage and all other connective tissue.

Thermal induction of scar collagen deposition is a non-ablative neosynthetic process of collagen deposition as a reaction to inflammation induced by thermal injury. The resulting scar collagen is frequently referred to as nascent, as opposed to pre-existing.

Standard thermal gradient is the thermal content of tissue that is greater on the skin surface.

Reverse thermal gradient is, (i) the application of electromagnetic energy to alter the biophysical properties of collagen, i.e., contraction, with minimal blistering of the tissue surface, (ii) a gradient in which the tissue surface temperature is cooler than the underlying collagen tissue, (iii) conditions in which a standard thermal gradient is reduced or equalized in temperature between the tissue surface and the underlying collagen, or (iv) monitoring the heat content (temperature and exposure duration) of the tissue surface to avoid blistering during treatment, regardless of the tissue surface temperature relative to the underlying collagen tissue.

Transcutaneously means that the delivery device delivers electromagnetic energy directly through the tissue surface.

Percutaneously means that the delivery device is inserted through the skin or the tissue surface through an endoscope, arthroscope, and the like.

Transmucosal means that the delivery device delivers electromagnetic energy directly through the mucosal surface.

Permucosal means that a delivery device is inserted through a mucosal surface through an endoscope, arthroscope, and the like.

A first degree burn means a burn that involves only the epidermis. It is characterized by erythema.

A second degree burn means a burn that destroys the epithelium and a variable portion of the dermis.

A third degree burn means a burn that destroys the entire thickness of skin, including the epithelium and dermis.

The present invention provides, (i) a method for forming and contracting scar collagen below a tissue surface in a selected tissue site, (ii) a method for forming callus deposition in a selected periosteum tissue site, (iii) an apparatus for applying radiant energy from a thermal energy source to underlying collagen tissue without substantially modifying the melanocytes and other epithelial cells found in the epidermis and (iv) an apparatus for applying radiant energy to underlying subcutaneous or deeper soft tissue layers that include loculations of fat with fibrous septae made of collagen tissue.

The methods of the present invention do not provide for total necrosis of cells. Instead, with the method and apparatus creating the desired contour effect, the loculations of fat with the fibrous septae made of collagen tissue use a reverse thermal gradient applied to the underlying collagen tissue layers resulting in a partial denaturization of the collagen permitting it to become tightened. This is achieved without killing all of the fat cells within the loculations.

The formation or induction of scar collagen formation can be done transcutaneously, with a reverse thermal gradient, percutaneously, transmucosally, permucosally, or through a device including but not limited to an endoscope. An electromagnetic energy apparatus is provided and includes an electromagnetic energy source and a delivery device. The delivery device is positioned on the tissue surface. Electromagnetic energy is produced from the electromagnetic energy source and delivered through the tissue surface to the selected tissue site for a sufficient time to induce scar collagen formation in the selected tissue site. No deeper than a second degree burn is formed on the tissue surface. The scar collagen is subsequently contracted. This method is particularly useful in soft tissue sites that are devoid or deficient in collagen.

An electromagnetic energy apparatus is provided and includes an electromagnetic energy source and a delivery device. The delivery device is positioned on a tissue surface of the selected periosteum tissue site. Electromagnetic energy is produced from the electromagnetic energy source. Electromagnetic energy is delivered from the delivery device, through the tissue surface, and to the selected periosteum tissue site for a sufficient time to induce callus formation in the selected periosteum tissue site. The callus is subsequently contracted. The method for forming callus can be done transcutaneously, with a reverse thermal gradient, percutaneously, transmucosally permucosally, or through a device including but not limited to an endoscope.

The methods of the present invention use an electromagnetic energy source to apply electromagnetic energy to a selected tissue site. The electromagnetic energy can be delivered transcutaneously, with a reverse thermal gradient, percutaneously, transmucosally, permucosally, or through a device including but not limited to an endoscope. The electromagnetic energy induces scar collagen formation in tissue sites that, (i) have pre-existing collagen, (ii) are deficient in pre-existing collagen, or (iii) lack pre-existing collagen. Following the formation of the scar collagen, the application of electromagnetic energy contracts the scar collagen.

Additionally, the methods of the present invention provide for the contraction of collagen tissue underlying a tissue surface area. The overlying layer of tissue is not ablated. No deeper than a second degree burn is produced in the overlying layer of tissue, and preferably no deeper than a first degree burn.

Suitable applications for the methods of the present invention include but are not limited to, tightening and firming soft tissue, treatment of unstable joints due to collateral ligament laxity, the treatment of unstable spinal column disorders, treatment of weaknesses of the abdominal wall, treatment of other connective tissues, esophageal hernia with reflux, urinary incontinence in women, dysdynamic segments of the myrocardium and other aneurysmal dilatations of the vessels, sleep apnea, laxity and wrinkling of the skin, and the like.

Laxity and wrinkling of the skin occurs as a consequence of inadequate support of the epidermis. The induction of scar collagen deposition is used for the treatment of wrinkles. Improved skin turgor is accomplished by first replenishing the collagen matrix that has been lost with aging. Following the deposition of nascent scar collagen in the dermis, contraction of collagen with a reverse thermal gradient corrects the laxity and wrinkling of the skin without resorting to resurfacing techniques that require the application of a standard thermal gradient burn to the skin. This is achieved without undergoing a lengthy post-operative healing process. Bleeding and infection are reduced. Second degree burns to the superficial skin are minimized. The melanocytes are not damaged and pigmentary irregularities are avoided.

In one embodiment, skin tightening with a reverse thermal gradient contraction of collagen corrects areas such as the thighs, knees, arms, back, face and neck lifts, and hips without unsightly scarring. Elastosis, or stretching of the abdominal skin from pregnancy is corrected without the long scar commonly associated with an abdominoplasty. Breast uplifts, i.e., mastoplexies, no longer require extensive incisions.

Thermal remodeling of collagen can occur with both native (dermal) collagen and collagen produced as part of the healing process. Wound healing involves an initial inflammatory stage that is followed by a period of rapid nascent collagen production that morphologically appears as a scar. The biophysical properties of collagen are the same regardless of its origin.

One apparatus used to create the reverse thermal gradient is a composite heating pad that has both cooling elements and electromagnetic delivery devices. The heating pad is configured to the topography of the treatment area and is incorporated into an elastic garment. Partial denaturization of collagen is achieved with each treatment. Thermal transducers measure the surface temperature of the treatment area to avoid blistering. In one embodiment the deeper dermis is heated to above 65 degrees for collagen contraction. Sequential treatments are designed to allow for more precision of the end result. Areas of application are not confined by requirements to either hide surgical incisions or transition along aesthetic boundaries.

Various types of electromagnetic energy can be utilized with the present invention. Electromagnetic energy may be any kind that can cause cell heating or physical destruction by being applied to collagen tissue. Examples of suitable electromagnetic energy sources include, but are not limited to RF, microwave, ultrasound, laser and the like.

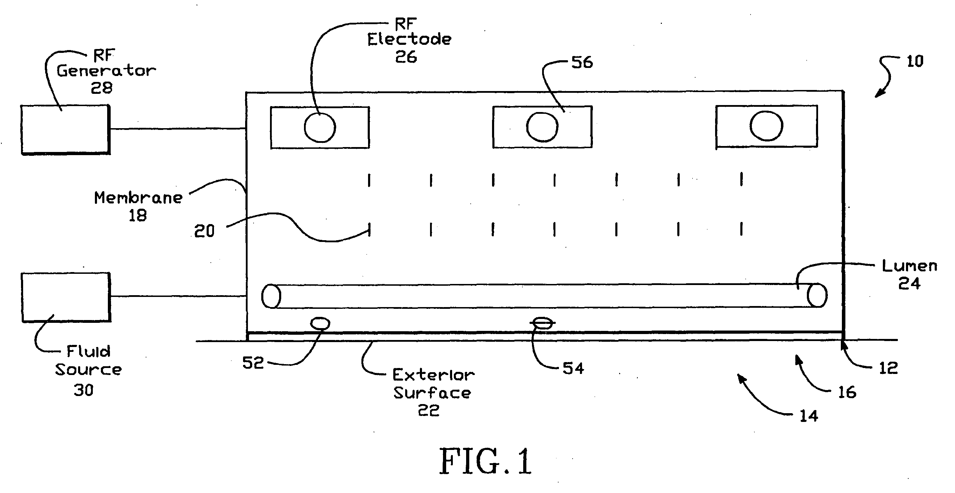

Referring now to Fig. 1, an apparatus 10 applies electromagnetic energy through a skin layer 12, such as the epidermis, and to the underlying collagen tissue 14 without substantially modifying melanocytes and other epithelial cells 16 found in the lower layer of epidermis layer 12.

A porous membrane 18 is adapted to receive an electrolytic solution 20. Porous membrane 18 becomes inflated to substantially conform a contacting exterior surface 22 of porous membrane 18 which is in close thermal contact with epidermis 12. Porous membrane 18 includes a cooling lumen 24 for receiving a cooling fluid that imparts a cooling effect on epidermis layer 12.

One or more electromagnetic electrodes 26 are positioned at various places in porous membrane 18. In one embodiment, electromagnetic electrodes 26 are positioned on a side that is substantially opposing to contacting exterior surface 22. In other embodiments, electromagnetic electrodes 26 are placed closer to cooling lumen 24. In embodiment particularly suitable for the hips, porous membrane is about 20 cm by 30 cm, with an oval shape.

An electromagnetic power source 28 is coupled to electromagnetic electrodes 26 and a source of electrolytic solution 30 is coupled to porous membrane 18.

In one method of the present invention, collagen tissue in a dermis underlying the epidermis of the skin is transcutaneously contracted with the use of a thermal heating apparatus. Electromagnetic energy is transcutaneously delivered through the epidermis to the underlying dermis. Fibroblast proliferation is initiated in the underlying dermis. Scar collagen is formed in the underlying dermis. The scar collagen is subsequently contracted and the skin is tightened.

In another embodiment, a method is provided for contracting collagen tissue in a subcutaneous fat layer through an overlying epidermis layer. A thermal heating apparatus produces electromagnetic energy. The electromagnetic energy can be delivered transcutaneously, with a reverse thermal gradient, percutaneously, transmucosally permucosally, or through a device including but not limited to an endoscope. The electromagnetic energy is directed through the epidermis to the underlying subcutaneous fat layer. Fibroblast proliferation is initiated in the subcutaneous fat layer. Scar collagen is formed and then tightened.

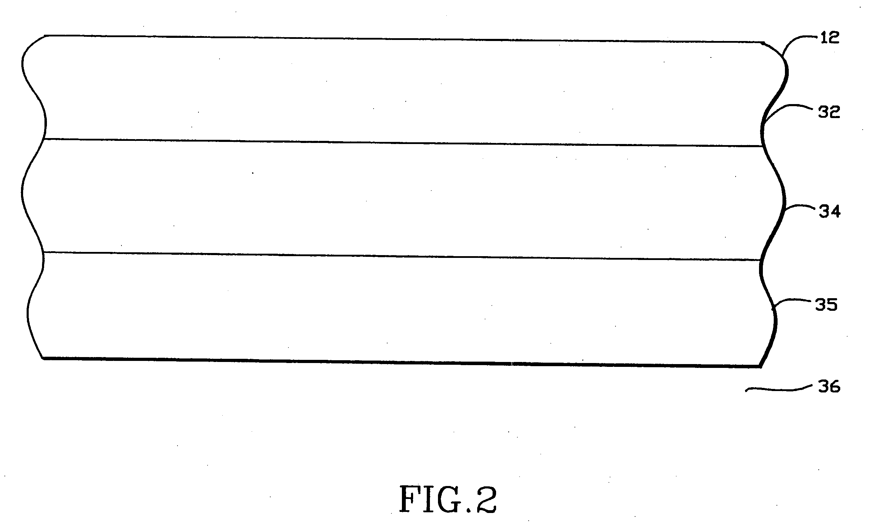

With referenced now to Fig. 2, electromagnetic energy can be applied through epidermis layer 12, to papillary dermis layer 32, to reticular dermis layer 34, to subcutaneous layer 35, as well as to underlying soft tissue 36. The extent of collagen in the various layers is < 5% in the epidermis, ∼ 50% in the dermis, ∼ 20 % in the subcutaneous, ∼ 10% in the muscle with overlying fascia. Shrinking of collagen tissue takes place in a direction parallel to the axis of the collagen fibers. Thermal shrinkage of collagen begins with the denaturization of the triple helix structure of the collagen fibers. This occurs when electromagnetic energy is applied to the collagen tissue causing the hydrolysis of heat labile cross links of the collagen network.

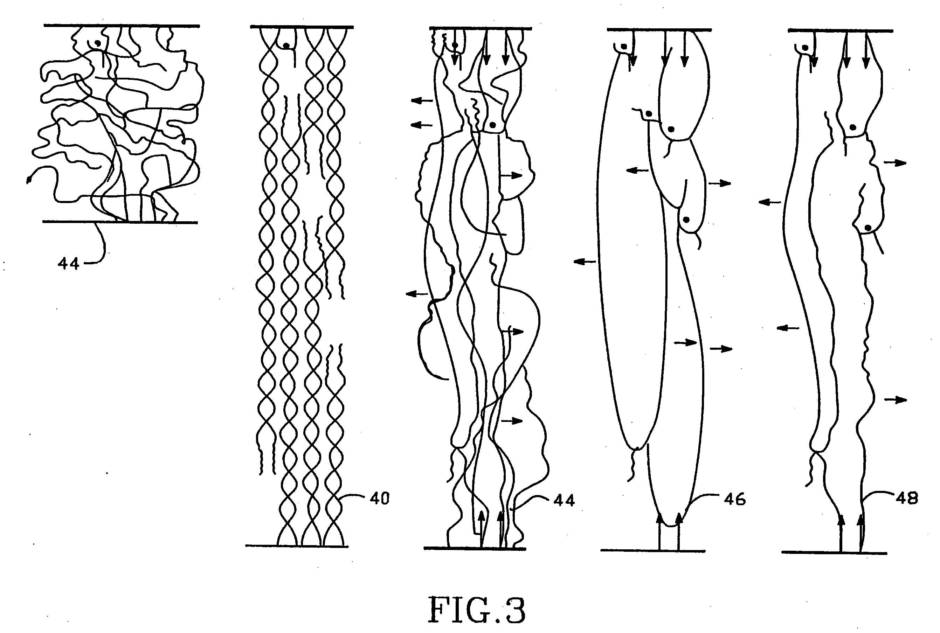

Fig. 3 is a schematic representation of a collagen network behavior under the influence of heat The thickened lines represent the chains originally bound by covalent cross links. The arrows indicate tensions exerted on the collagen chains by the effect of heat More particularly, Fig. 3 illustrates (i). native collagen network 40, (ii). collagen 42 under isometric conditions, (iii). collagen network without any restraint, (iv). collagen network 46 under isometric tension as long as the nodes are stable, and (v). collagen network 48 under isometric tension after some cross links have been cleaved.

Electromagnetic electrodes 26 can be RF electrodes comprising a single electrode, or a plurality which can form a segmented flexible circuit. Electromagnetic power source 28 is then an RF generator. Electrolytic solution 20 is introduced into porous membrane 18 and passes by RF electrodes 26. Electrolytic solution 20 transfers RF power from RF electrodes 28 to the desired underlying collagen tissue to achieve partial denaturization of the collagen molecule.

Generally, RF electrodes 26 can be monopolar or bipolar. In the monopolar mode, RF current flows through body tissue from a return electrode which can be in a form of a conductive pad applied to the patients outer skin. Maximum heating occurs where the current density is the greatest.

During a treatment phase, the denaturization of collagen molecules can be conducted under feedback control. Treatment can occur without the attention of medical supervision. Feedback is accomplished by (i). visualization, (ii). impedance, (iii). ultrasound, or (iv). temperature measurement. Optionally included and preferably positioned on contacting exterior surface 22 can be one ore more thermal sensors 52, as well as one or more impedance monitors 54. Thermal sensors 52 permit accurate determination of the surface temperature of epidermis layer 12.

Electrolytic solution 20 can be preheated to a selected temperature and modified as necessary. This reduces the amount of time needed to effect at satisfactory denaturization of collagen molecules and subsequent skin tightening.

Porous membrane 18 can be made of a material that is an insulator. For purposes of this disclosures, an insulator is a barrier to thermal or electrical energy flow. Porous membrane 18 can be made of a material which permits controlled delivery of electrolytic solution 20 to epidermis layer 12. Porous membrane 18 can be made of a variety of materials including, but not limited to knitted polyester, continuous filament polyester, polyester-cellulose, rayon, polyamide, polyurethane, polyethylene and the like. Suitable commercial products include, (i). Opcell available from Centinal Products Corp., Hyannis, Mass, and (ii). UltraSorb, HC 4201 or HT 4644 MD from Wilshire Contamination Control, Carlsbad, California. Pockets or zones 56 can be formed around RF electrodes 26. Each pocket 56 has a lower porosity for the flow of electrolytic solution 20 than all other sections of porous membrane 18. Differences in porosity can be achieved with different types of materials which form porous membrane 18. Electrolytic solution 20 is retained in pockets 56 longer than in non-pocket sections of porous membrane 18, and there is a greater transfer of RF energy to electrolytic solution 20, creating a larger electrode. The larger electrode produces RF and thermal energy to create a larger electrode effect. However, this does not effect the creation of the reverse thermal gradient. RF energy is still transferred through porous membrane 18 passing in the vicinity of cooling lumen 24, in order to create a lower temperature at epidermis layer 12 and the temperature increases as deeper layers are reached.

In a skin contracting method of the present invention, a tighter, more youthful skin envelope is achieved. This is accomplished without undergoing a lengthy post-operative healing process. Bleeding and infection is reduced. Second degree burns to the superficial skin are minimized. The melanocytes are not damaged and pigmentary irregularities are avoided.

Because scarring and pigmentary irregularities are avoided, skin or other tightening occurs in areas previously considered "off-limits" to standard methods of surgical resection, laser and chemical resurfacing. Skin tightening with a reverse thermal gradient contraction of collagen can correct areas including but not limited to the thighs, knees, arms, back and hips without unsightly scarring of standard techniques. In addition, areas previously corrected by aesthetic procedures, such as face and neck lifts, can be corrected without requiring surgery or the typical incisions around the ear. Elastosis or stretching of the abdominal skin from pregnancy can be corrected without the extensive incision of an abdominoplasty. The method of the present invention can also be used for mastopexies or breast uplifts..

The fibrous septae in subcutaneous fat layers can be contracted to tighten the soft tissue. Along with these extracellular effects of collagen, intracellular thermal induction effects upon the fat cell or lipocyte results in a net egress of fat from the lipocyte which achieves a net reduction of volume of the treated area. A second thermal device is used in tandem with the initial thermal device to achieve liposculpture of the treated area. The second device can be designed with a convergent lens that is focused at the appropriate level on the subcutaneous tissue.

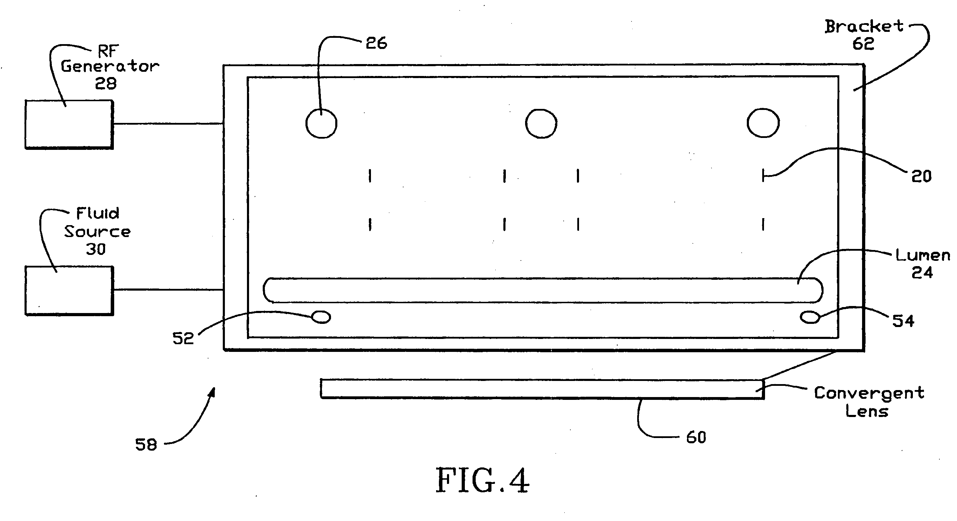

Referring now to Fig. 4, an apparatus 58 for creating a desired contour effect of underlying subcutaneous layers or deeper soft tissue layers which include loculations of fat with fibrous septae made of collagen tissue is illustrated. The apparatus 58 of Fig 4, includes a porous membrane 18, electrolytic solution 20, a contacting exterior surface 22, a cooling lumen, electromagnetic electrodes 26, an electromagnetic power source 28, an electrolytic solution source 30, one or more thermal sensors 52, as well as one or more impedance monitors 54. Apparatus 58 also includes a focussing element 60 which focuses electromagnetic energy from electrolytic solution 20 to the underlying collagen tissue. Focussing element 60 and electrolytic solution 20 create a reverse thermal gradient from epidermis layer 12 to the underlying collagen tissue 14. Focussing element 62 can be, in the case of ultrasonic energy, a lens having a flat planer surface on the radiation wave incident side and a concave exit face, see *Ultrasonics Theory and Application,* by G.L. Goberman, Heart Publishing Co., New York (1959), at section 2.6. The use of such a focussing lens for ultrasonic energy with a planer wave receiving face and concave exit face is also described in the article "Deep Local Hypothermia for Cancer Therapy: Extreme Electromagnetic and Ultrasound Technics," A.Y. Cheung and A. Neyzari, *Cancer Research,* Vol. 44, pp.4736-4744, October 1984.

Rf can be the electromagnetic energy source, and various localizing techniques, well known in the art, can be utilized. In one embodiment, radio frequency energy is supplied by capacitive coupling directly to epidermis layer 12 for areas close to the dermal tissue. Radio frequency induction focussing can be achieved with the use of plural focussing coils which are adaptive at the zone of interest and are elsewhere subtractive. Alternatively, radio frequency energy may be focused by having a multiple beam phased array. For concave focussing see, "Tumor reduction by radio frequency therapy response", H.H. LeVeen et al., *JAMA,* Vol. 233, at 2198-2200.

Alternative radio frequency focussing methods are disclosed in "Equipment for Local Hypothermia Therapy of Cancer", C.F. Babbs et al., *Medical Instrumentation,* Vol. 16, No. 5, Sept-Oct 1982, pp.245-248.

It will be appreciated that focussing element 60 can be a convergent lens. Further, focussing element 60 can be positioned in porous membrane 18, and at the exterior 16 between epidermis layer 12 and porous membrane 18. Further, a coupling device 62 can be included which couples focussing element 60 with porous membrane 18. In one embodiment, coupling device 62 is a bracket which is positioned around a periphery of porous membrane 18, and supports focussing element 50 in relation to porous membrane 18.

In the method for tightening skin, porous membrane 18 and thermal energy source 26 are provided. A reverse thermal gradient is created which cools a surface of epidermis layer 12 while heating underlying collagen containing layers. Epidermis layer 12 as well as underlying collagen containing tissue are heated, without substantially effecting the melanocytes and other epithelial cells in epidermis layer 12, resulting in a denaturization of collagen molecules, causing a contraction of the collagen tissue and a tightening of the skin. This method can be applied numerous times. In many instances, it may be desirable to tighten the skin to a certain level and then in subsequent treatments the skin is tightened further. There may be four fine treatments to fine tune the contour effects with greater precision. In this method, collagen containing tissue is partial denatured and fat cell destruction is minimized. This is achieved by partially denaturing by cleaving heat labile cross links of the collagen molecules.

The reverse thermal gradient provides a variation in temperature throughout the various tissue layers. For example, in various embodiments, the reverse thermal gradient has a tissue surface temperature range from about 40 to 60 degrees C, and a selected underlying tissue site temperature, i.e., where scar collagen is formed or where collagen is contracted, of about 60 to 80 degrees C. In other embodiments, when the reverse thermal gradient is a diminished or equalized standard thermal gradient the temperature ranges can be much broader.

In another embodiment, a method for liposculpturing an area of the body where there is an underlying area comprised of a loculation of fat that has collagen tissue as a fibrous septae also includes creating a reverse thermal gradient from epidermis layer 12 to the desired underlying loculation of fat layer. Sufficient electromagnetic energy is supplied through epidermis layer 12, without damaging or substantially modifying the melanocytes and other epithelial cells, through other skin layers and is focused on the collagen tissue of the fibrous septae. Electromagnetic energy partially denatures the collagen tissue with a minimal destruction of fat cells. Again, this is achieved by partially denaturizing, e.g., by cleaving, heat labial cross links of collagen molecules. The reverse thermal gradient produces a net mobilization of intra-cellular fat with diminished destruction of fat cells.

In yet another embodiment of the invention, thermal induction of osteoblasts in the periosteum results in callus (calcium matrix) deposition. Callus contains a higher percentage of collagen than mature bone and subsequent remodeling with thermal contraction is possible. Maturation of the remodelled callus with calcium deposition results in stable bony fusion of treated areas.

Without limitation, power source 28 can be an RF source. RF power source 28 feeds energy to an RF power generator 64 and then to RF electrodes 26. A multiplexer 66 measures current, voltage and temperature, at the numerous thermal sensors associated with to each RF electrode 26. RF electrodes 26 can be individually measured. Multiplexer 66 is driven by a controller 68 which can be a digital or analog controller, or a computer with software. When controller 68 is a computer it can include a CPU coupled through a system bus. On the system can be a keyboard, disk drive, or other non volatile memory systems, a display, and other peripherals, as are well known in the art. Also coupled to the bus are a program memory and a data memory.

An operator interface 70 includes operator controls 72 and a display 74. Controller 68 can be coupled to different types of imaging systems including ultrasonic, thermal sensors 52, and impedance monitors 54.

Current and voltage are used to calculate impedance. A diagnostic phase can be initially run to determine the level of treatment activity. This can be done through ultrasound as well as other means. Diagnostics can be performed both before and after treatment.

Thermal sensors 52, and thermal sensors 76 contained within RF generator 64 measure voltage and current that is delivered to the desired treatment site. The output for these sensors is used by controller 68 to control the delivery of RF power. Controller 68 can also control temperature and power. An operator set level of power and/or temperature may be determined and this will not be exceeded. Controller 68 maintains the set level under changing conditions. The amount of RF energy delivered controls the amount of power. A profile of power delivered can be incorporated in controller 68, as well as a preset amount of energy to be delivered. Feedback can be the measurement of impedance, temperature, or other indicators and occurs either at control 68 or at RF generator 64, if it incorporates a controller. For impedance measurement, this can be achieved by supplying a small amount of non therapeutic RF energy. Voltage and current are then measured to confirm electrical contact.

Circuitry, software and feedback to controller 68 result in full process control and are used to change, (i). power, (ii). the duty cycle, (iii). monopolar or bipolar energy delivery, (iv). electrolytic solution 20 delivery, flow rate and pressure and (v). can determine when the process is completed through time, temperature and/or impedance. These process variables can be controlled and varied based upon tissue temperature monitored at multiple sites on contacting exterior surface 22 as well as monitoring impedance to current flow at each RF electrode 26, indicating changes in current carrying capability of the tissue during the process. Further, controller 68 can provide multiplexing, monitor circuit continuity, and determine which RF electrode 26 is activated.

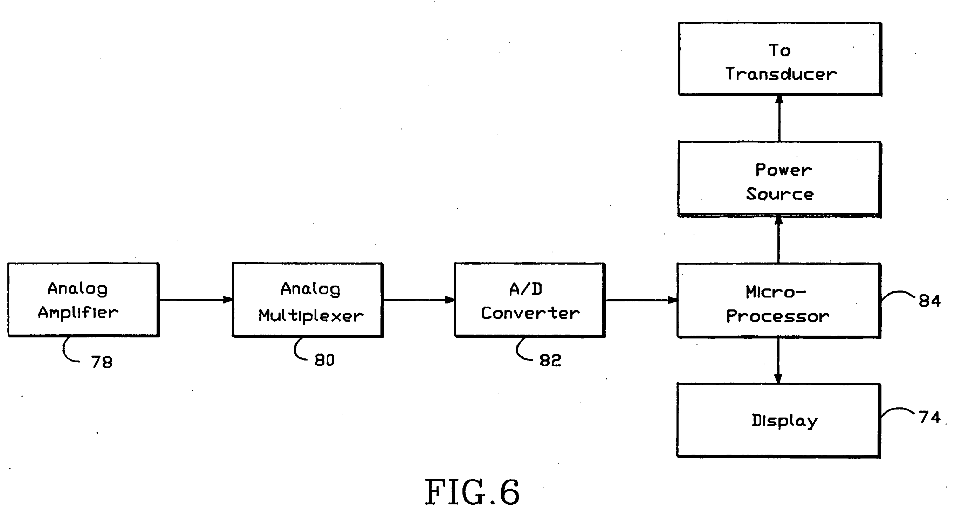

A block diagram of one embodiment of suitable processing circuitry is shown in Fig. 6. Thermal sensors 52 can be thermistors which have a resistance that varies with temperature. Analog amplifier 78 can be a conventional differential amplifier circuit for use with thermistors and transducers. The output of analog amplifier is sequentially connected by an analog multiplexer 80 to the input of an analog digital converter 82. The output of amplifier 78 is a voltage which represents the respective sensed temperatures. The digitized amplifier output voltages are supplied by analog to digital converter 82 to a microprocessor 84. Microprocessor 84 calculates the temperature or impedance of the tissue. Microprocessor 84 can be a type 6800. However, it will be appreciated that any suitable microprocessor or general purpose digital or analog computer can be used to calculate impedance or temperature.

Microprocessor 84 sequentially receives and stores digital representations of impedance and temperature. Each digital value received by microprocessor 84 corresponds to different temperatures and impedances.

Calculated temperature and impedance values can be indicated on display 74. Alternatively, or in addition to the numerical indication of temperature or impedance, calculated impedance or temperature values can be compared by microprocessor 84 with temperature and impedance limits. When the values exceed predetermined temperature or impedance values a warning can be given on display 74 and additionally, the delivery of RF energy to its respective electrode can be decreased or multiplexed to another electrode. A control signal from microprocessor 84 can reduce the power level by RF generator 64, or de-energize the power delivered to any particular electrode. Controller 68 receives and stores the digital values which represent temperatures and impedances sent. Calculated surface temperatures and impedances can be forwarded by controller 68 to display 74. If desired, the calculated surface temperature of epidermis layer 12 is compared with a temperature limit and a warning signal can be sent to display 74. Similarly, a control signal can be sent to RF power source 26 when temperature or impedance values exceed a predetermined level.

The foregoing description of a preferred embodiment of the invention has been presented for purposes of illustration and description. It is not intended to be exhaustive or to limit the invention to the precise forms disclosed. Obviously, many modifications and variations will be apparent to practitioners skilled in this art. It is intended that the scope of the invention be defined by the following claims and their equivalents.

## Claims

1. An apparatus for delivering thermal energy through an external skin surface to an underlying collagen containing tissue, comprising:
thermal delivery means including an interface surface configured to conform to the exterior skin layer surface;
electrode means coupled to the thermal delivery means and configured to transfer thermal energy through the interface surface and the skin layer surface to the underlying collagen tissue;
thermal energy control means coupled to the thermal delivery means and configured to provide sufficient thermal energy from the electrode means to contract the underlying collagen tissue with no deeper than a first degree burn formed on the exterior skin layer surface; and
cabling means coupled to the electrode means.

2. The apparatus of claim 1, wherein the electrode means is an RF electrode coupled to an RF power source.

3. The apparatus of claim 1, further comprising:
a thermal energy source coupled to the electrode means.

4. The apparatus of claim 1, wherein the electrode means is an ultrasound emitter coupled to an ultrasound energy source.

5. The apparatus of claim 1, wherein the thermal energy control means comprises:
a cooling channel positioned in an interior of the thermal energy delivery means and coupled to a source of a cooling medium.

6. The apparatus of claim 1, wherein the thermal delivery means is a membrane.

7. The apparatus of claim 6, wherein the an interior of the membrane is configured to contain an electrolytic solution.

8. The apparatus of claim 1, wherein the thermal energy control means is configured to form a reverse thermal gradient at the exterior skin surface with a temperature range of 30 degrees C to 80 degrees C from the external skin surface to the underlying collagen containing tissue.

9. The apparatus of claim 1, further comprising:
one or more thermal sensors positioned on the interface surface.

10. The apparatus of claim 1, further comprising:
a feedback device coupled to the electrode means responsive to a detected characteristic of the external skin surface or the underlying collagen containing tissue.

11. A cosmetic method for tightening skin, comprising:
providing a skin tightening apparatus including an electrode means for delivering thermal energy through a skin tightening apparatus interface surface and an external skin surface to an underlying collagen containing tissue;
positioning the skin tightening apparatus interface surface adjacent to the external skin surface;
delivering sufficient thermal energy from the electrode means through the external skin surface to contract the underlying collagen tissue with no deeper than a first degree burn formed on the exterior skin layer surface; and
tightening the external skin surface.

12. The method of claim 11, wherein the delivery of thermal energy at least partially denatures the collagen containing tissue by cleaving heat labile cross-links of collagen molecules.

13. The method of claim 12, wherein the delivery of thermal energy at least partially denatures the collagen containing tissue while minimizing cellular destruction.

14. The method of claim 12, wherein the delivery of thermal energy at least partially produces a net mobilization of intracellular fat with a diminished destruction of cells.

15. The method of claim 11, wherein the electrode means is an RF electrode coupled to an RF power source.

16. The method of claim 11, wherein the electrode means is an ultrasound transmitter coupled to an ultrasound energy source.

17. The method of claim 1, wherein sufficient thermal energy is delivered to contract the underlying collagen tissue with no deeper than a second degree bum formed on the exterior skin layer surface

18. The method of claim 11, wherein the collagen containing tissue is in a subdermal layer.

19. The method of claim 11, wherein the collagen containing tissue is in a dermal layer.

20. The method of claim 11, wherein the collagen containing tissue is in a subcutaneous layer.

21. The method of claim 11, wherein the collagen containing tissue is in fascial and muscle tissue.

22. A cosmetic method for forming and contracting scar collagen below an external skin surface, comprising:
providing a scar collagen apparatus including an electrode means for delivering thermal energy through a scar collagen apparatus interface surface and the external skin surface to an underlying tissue site;
positioning the scar collagen apparatus interface surface on the external skin surface; and
delivering sufficient thermal energy from the electrode means to the underlying tissue site to induce scar collagen formation in the selected tissue site with no deeper than a first degree burn formed on the exterior skin layer surface.

23. The method of claim 22, wherein sufficient thermal energy is delivered to induce scar collagen formation in the selected tissue site with no deeper than a second degree burn formed on the external skin layer surface.

24. The method of claim 22, wherein the thermal energy is transcutaneously delivered to the underlying tissue site.

25. The method of claim 22, wherein the thermal energy is percutaneously delivered to the underlying tissue site.

26. The method of claim 25, wherein the electrode means is a laser delivery device coupled to a laser source.

27. The method of claim 22, wherein the thermal energy is transmucosally delivered to the underlying tissue site.

28. The method of claim 22, wherein the thermal energy is permucosally delivered to the underlying tissue site.

29. The method of claim 22, wherein the thermal energy is transcutaneously delivered to the underlying tissue site for a sufficient time to induce scar collagen formation in the underlying tissue site with no deeper than a first degree burn formed on the tissue surface.

30. The method of claim 22, wherein the thermal energy is transcutaneously delivered to the underlying tissue site for a sufficient time to induce scar collagen formation in the underlying tissue site with no deeper than a second degree burn formed on the tissue surface.

31. The method of claim 22, wherein the underlying tissue site is substantially devoid of collagen.

32. The method of claim 22, wherein the underlying tissue site is deficient of collagen.

33. The method of claim 22, wherein the underlying tissue site has pre-existing collagen.

34. The method of claim 22, wherein the external skin surface is heated to a temperature range of 40 to 60 degrees C.

35. The method of claim 22, wherein the underlying tissue site is heated to a temperature of 60 to 80 degrees or greater.

36. A cosmetic method for contracting collagen below a tissue surface in a selected tissue site, comprising:
providing a collagen contraction apparatus including an electrode means for delivering thermal energy through a collagen contraction apparatus interface surface and an external skin surface to the selected tissue site;
positioning the collagen contraction apparatus interface surface on the external skin surface; and
delivering sufficient thermal energy from the electrode means to the selected tissue site for a sufficient time to at least partially contract collagen in the selected tissue site with no deeper than a first degree burn formed on the exterior skin layer surface.

37. The method of claim 36, wherein sufficient thermal energy is delivered to induce scar collagen formation in the selected tissue site with no deeper than a second degree burn formed on the external skin layer surface.

38. The method of claim 36, wherein the thermal energy is transcutaneously delivered to the underlying tissue site.

39. The method of claim 36, wherein the thermal energy is percutaneously delivered to the underlying tissue site.

40. The method of claim 36, wherein the electrode means is a laser delivery device coupled to a laser source.

41. The method of claim 36, wherein the thermal energy is transmucosally delivered to the underlying tissue site.

42. The method of claim 36, wherein the thermal energy is permucosally delivered to the underlying tissue site.

43. The method of claim 36, wherein the thermal energy is transcutaneously delivered to the underlying tissue site for a sufficient time to induce scar collagen formation in the underlying tissue site with no deeper than a first degree burn formed on the tissue surface.

44. The method of claim 36, wherein the thermal energy is transcutaneously delivered to the underlying tissue site for a sufficient time to induce scar collagen formation in the underlying tissue site with no deeper than a second degree bum formed on the tissue surface.

45. The method of claim 36, wherein the underlying tissue site is substantially devoid of collagen.

46. The method of claim 36, wherein the underlying tissue site is deficient of collagen.

47. The method of claim 36, wherein the underlying tissue site has pre-existing collagen.

48. The method of claim 36, wherein the external skin surface is heated to a temperature range of 40 to 60 degrees C.

49. The method of claim 36, wherein the underlying tissue site is heated to a temperature of 40 to 80 degrees or greater.

50. A cosmetic method for forming callus deposition in a selected periosteum tissue site, comprising:
providing a callus deposition apparatus including an electrode means for delivering thermal energy through a callus deposition apparatus interface surface and an external skin surface to the selected periosteum tissue site;
positioning the collagen contraction apparatus interface surface on the external skin surface; and
delivering sufficient thermal energy from the electrode means to the selected periosteum tissue site for a sufficient time to form callus deposition in the selected periosteum tissue site with no deeper than a first degree burn formed on the exterior skin layer surface.

51. The method of claim 50, wherein the thermal energy is transcutaneously delivered to the selected periosteum tissue site.

52. The method of claim 50, wherein the thermal energy is percutaneously delivered to the selected periosteum tissue site.

53. The method of claim 52, wherein the electrode means is a laser delivery device coupled to a laser source.

54. The method of claim 50, wherein the thermal energy is transmucosally delivered to the selected periosteum tissue site.

55. The method of claim 50, wherein the thermal energy is permucosally delivered to the selected periosteum tissue site.

56. The method of claim 50, wherein the exterior skin surface is heated to a temperature range of 40 to 60 degrees C.

57. The method of claim 50, wherein the selected periosteum tissue site is heated to a temperature of 60 to 80 degrees or greater.

58. The method of claim 50, wherein the electromagnetic energy forms the callus with no more than a first degree burn formed on the surface of the selected periosteum tissue site.

59. The method of claim 50, wherein the electromagnetic energy forms the callus with no more than a second degree burn formed on the surface of the selected periosteum tissue site.

60. An apparatus for delivering thermal energy through an exterior skin surface to an underlying tissue site including loculations of fat with fibrous septae made of collagen tissue, comprising:
to create a desired contour effect without substantially modifying melanocytes in the epidermis, comprising:
thermal delivery means including an interface surface configured to conform to the exterior skin layer surface;
electrode means coupled to the thermal delivery means and configured to transfer thermal energy through the interface surface and the exterior skin layer surface to the underlying tissue site;
thermal energy control means coupled to the thermal delivery means and configured to provide sufficient thermal energy from the electrode means to the underlying tissue site to contract the fibrous septae and create a desired contour effect with no deeper than a first degree burn formed on the exterior skin layer surface; and
cabling means coupled to the electrode means.

61. The apparatus of claim 60, wherein the electrode means is an RF electrode coupled to an RF power source.

62. The apparatus of claim 60, further comprising:
a thermal energy source coupled to the electrode means.

63. The apparatus of claim 60, wherein the electrode means is an ultrasound emitter coupled to an ultrasound energy source.

64. The apparatus of claim 60, wherein the thermal energy control means comprises:
a cooling channel positioned in an interior of the thermal energy delivery means and coupled to a source of a cooling medium.

65. The apparatus of claim 60, wherein the thermal delivery means is a membrane.

66. The apparatus of claim 65, wherein an interior of the membrane is configured to contain an electrolytic solution.

67. The apparatus of claim 60, wherein the thermal energy control means is configured to form a reverse thermal gradient at the exterior skin surface with a temperature range of 30 degrees C to degrees C from the external skin surface to the underlying tissue site.

68. The apparatus of claim 60, further comprising:
one or more thermal sensors positioned on the interface surface.

69. The apparatus of claim 60, further comprising:
a feedback device coupled to the electrode means responsive to a detected characteristic of the external skin surface or the underlying collagen containing tissue.

70. A cosmetic method of sculpturing an underlying tissue site made of a loculation of fat including a collagen containing tissue as a fibrous septae, comprising:
providing a tissue sculpturing apparatus including an electrode means for delivering thermal energy through a tissue sculpturing apparatus interface surface and an external skin surface to the underlying tissue site;
positioning the tissue sculpturing apparatus interface surface adjacent to the external skin surface;
delivering sufficient thermal energy from the electrode means to the underlying tissue site to contract the collagen containing tissue with no deeper than a first degree burn formed on the exterior skin layer surface; and
sculpturing the collagen contain tissue to a desired contour.

71. The method of claim 70, wherein the collagen containing tissue is partially denatured by cleaving heat labile cross-links of collagen molecules.

72. The method of claim 70, wherein the collagen containing tissue is partially denatured while minimizing cellular destruction.

73. The method of claim 70, wherein the delivery of thermal energy produces a net mobilization of intracellular fat with diminished destruction of cells.

74. The method of claim 70, wherein the electrode means is an RF electrode coupled to an RF energy source.

75. The method of claim 70, wherein the electrode means is an ultrasound emitter coupled to an ultrasound energy source.

76. The method of claim 74, further comprising:
a source of electrolytic solution that delivers electrolytic solution to the RF electrode.

77. The method of claim 76, wherein RF energy is transferred from the RF electrodes to the electrolytic solution.

78. The method of claim 70, wherein the collagen containing tissue is in a subdermal layer.

79. The method of claim 70, wherein the collagen containing tissue is in a dermal layer.

80. The method of claim 70, wherein the collagen containing tissue is in a subcutaneous layer.

81. The method of claim 70, wherein the collagen containing tissue is in fascial and muscle tissue.
